# EUROPEAN PATENT APPLICATION

(11) **EP 0 652 528 A2**
(43) Date of publication of application: **10.05.1995**
(21) Application number: 94307860.0
(22) Date of filing: 26.10.1994
(51) Int. Cl.: G06F 19/00

(54) **Integrated computer system and method for processing medical informations and patient data**

(30) Priority: 05.11.1993 US 148050
(71) Applicant: AT&T Corp., New York, NY 10013-2412 (US)
(72) Inventor: Ahamed, Syed V., Somerset, New Jersey 08873 (US); Lawrence, Victor B., Holmdel, New Jersey 07733 (US)
(74) Representative: Watts, Christopher Malcolm Kelway, Dr.

(57) **Abstract**

The present invention involves a hospital-based integrated medical computer system for processing medical and patient information and for evolving medical knowledge, diagnoses and prognoses. It includes at least one processor including a memory and a plurality of medical data banks connected thereto, and a plurality of separate processor hardware modules at least indirectly connected to the memory. The modules include a communication module, at least one switching module, an administrative module and a knowledge base module. There is also hardware, firmware and software in the processor hardware modules to enable the modules to perform at least the following functions: for the communication module, to control all functional processes of the other modules, the main memory and the processor, so that they effectively communicate with one another; for the switching module(s), to select and switch between selected information as it becomes relevant to a process of solving a particular problem; for the administrative module, to perform housekeeping functions, including multitasking control with resource allocation, real-time multitasking and scheduling of tasks; and, for the knowledge base module, to operate knowledge processing functions and to store information in the medical data banks. In preferred embodiments, there are general patient databases, physician access point units, patient access point units, and service facilities. In other embodiments, a plurality of processors are included with their own memories and modules and are linked together to establish a processor unit. In processor net embodiments, there may be one or more separate integrated services digital networks dedicated to predetermined medical related functions.

## Description

### BACKGROUND OF THE INVENTION

### 1. Field of the Invention

The present invention relates to architecture for an integrated medical computer system and more specifically to a system utilizing one or more processors having separate modules for predetermined functions. Unlike data processing of conventional computer systems and unlike call processing switching systems, the present invention system is a significant improvement over modern electronic switching systems and includes separate modules for communication, switching and administration as, well as the addition of one or more knowledge modules coupled with a plurality of medical data banks.

### 2. Information Disclosure Statement

United States Patent No. 4,899,839 describes a method of monitoring a patient's medicine compliance. It involves weighing a container of medicine to determine a starting weight on a scale which is connected to a computer with a display unit and storing the starting weight in the computer followed by reweighing the container of medicine after a predescribed dosage is consumed to determine a second weight. The computer then determines the difference between the starting and said second weight to store a dosage unit weight. The computer is programmed to calculate compliance required weights of the container for each dosage administration for the prescription period of the medicine. The container of medicine is reweighed from time to time on the scale to compare actual weight with compliance required weight to determine compliance and the computer visually displays the compliance results on the display unit to permit compliance monitoring. Optionally, other patient characteristics are also monitored and feedback is provided.

United States Patent No. 5,016,172 is also directed to patient compliance and a status monitoring system. It involves utilizing an automatic compliance monitoring device which stores compliance information and which may be connected to a computer with a display unit. The compliance monitoring device or the computer is programmed to calculate compliance requirements of the container e.g. by number of cap openings, by dispensing count or by weight information obtained by the automatic compliance monitoring device, for each dosage administration for the prescription period. The automatic compliance monitoring device is periodically, occasionally, or randomly connected to the computer to compare actual usage with compliance required to determine compliance results on the display unit to permit compliance monitoring on a monitor at a remote location. Optionally, other patient characteristics are also monitored and feedback is provided.

While both of the above prior art patents utilize computer systems to provide patient monitoring information to a professional via a computer with feedback capabilities, they are limited by conventional computer architecture and do not teach or suggest the physical modules, integrated medical system processes, architecture and capabilities of the present invention system.

In conventional processing of data, the central processing unit plays the dominant role in executing the binary instructions in a pre-defined programmed sequence. Data availability and access is made feasible by the linking and loading functions. The processes involved in installing executable binary codes into the computer in usable form, are compiling, assembling and linking as well as the actual loading of the program and of the data into the core storage area of the computer. One process generally (and conveniently) forgotten by the computer scientists is the higher level language programming of a problem that is to be solved. Assuming no errors in these processes, the machine sequentially executes these instructions and brings the program to a normal termination and provides the user with the results that were being sought by the user without attention to the language of the program(s) in and loaded into the computer.

In telecommunication networks with electronic switching, the switching system or systems plays the dominant role in executing the various steps that are necessary for call processing. The sequence of the steps necessary for the completion of call processing is much more varied than the sequence of instructions for data processing. The switching systems may be distributed and the cooperative role of the various switching systems may become essential. This aspect is not unlike the controlled distribution of the processing in multiprocessor/multicomputer systems. Fortunately, with the evolution of the common channel interoffice signalling system and the standardization of its protocol, distributed call processing is not a problem in most modern communication networks. It is interesting to note that the level of programming in the switching systems is at higher level than the programming level of the third generation programming languages. This jump leaves the programmers of the switching systems with the more mundane functions of generating the executable code for the normal three modules (communication, switching, and administrative) of the switching system.

In modern "intelligent" networks (such is used by the Assignee herein, AT&T Corp., as well as other Universal Intelligent Networks), the service provisioning of the special services becomes the cooperative role of at least five well known interdependent computerized systems. These interdependent computerized systems are the service switching points, the service transfer points, the service control points, the service creation environment and intelligent peripherals.

Some of these known systems are substantially modified and/or supplemented in the present invention to create a unique integrated medical computer system environment. These significant changes are neither taught nor rendered obvious by the prior art.

### SUMMARY OF THE INVENTION

The present invention involves a hospital-based integrated medical computer system for processing medical and patient information and for evolving medical knowledge, diagnoses and prognoses. It includes at least one processor including a memory and a plurality of medical data banks connected thereto, and a plurality of separate processor hardware modules at least indirectly connected to the memory. The modules include a communication module, at least one switching module, an administrative module and a knowledge base module. There is also hardware, firmware and software in the processor hardware modules to enable the modules to perform at least the following functions: for the communication module, to control all functional processes of the other modules, the main memory and the processor, so that they effectively communicate with one another; for the switching module(s), to select and switch between selected information as it becomes relevant to a process of solving a particular problem; for the administrative module, to perform housekeeping functions, including multitasking control with resource allocation, real-time multitasking and scheduling of tasks; and, for the knowledge base module, to operate knowledge processing functions and to store information in the medical data banks. In preferred embodiments, there are general patient databases, physician access point units, patient access point units, and service facilities. In other embodiments, a plurality of processors are included with their own memories and modules and are linked together to establish a processor unit. In processor net embodiments, there may be one or more separate integrated services digital networks dedicated to predetermined medical related functions.

### BRIEF DESCRIPTION OF THE DRAWINGS

The present invention is more fully understood when the specification herein is taken in conjunction with the drawings appended hereto, wherein:
Figure 1 illustrates a schematic diagram of a present invention integrated medical computer system hospital-based medical processor unit with six buses for monitoring the flow of information in a large medical complex;
Figure 2 illustrates one embodiment of a present invention network based integrated medical computer system which has a plurality of processors, switching modules and knowledge base modules; and,
Figure 3 illustrates a simplified version of a present invention integrated medical computer system with limited functionality for a single input, single output and single user for system.

### DETAILED DESCRIPTION OF THE INVENTION

### I. Introduction

In the present invention integrated medical system, the processing is based upon the methodology fundamental to any problem solving. A problem is identified from the data at hand, additional search and confirmation is sought based upon a partial hypothesis or a hunch (intuitive pattern match). When a certain level of confidence is achieved, the existing knowledge banks are queried about categorizing the problem at hand. If the data is insufficient, an additional search is conducted to gain additional confirmation/denial of the partial hypothesis. Under conditions of certainty in the cause-effect relationship, fewer searches lead to the required confidence level before the solution is initiated. Under uncertainty or fear of persecution, more confirmation and consultation is sought regarding categorization, verifying the existing knowledge banks, and then the eventual solution to the problem accrues.

In the real world, these steps are iterated many times over, especially if there are numerous intertwined problems, numerous cause-effect relationships, and numerous remedies. Lack of complete input data causes additional uncertainty. Whereas every patient is unique in his/her own right, there is enough science and logic embedded in the profession that computers and networks can handle the various steps in the solution to the problem quickly, systematically, and within any given percentage of confidence. Apart from steps in the remedy and cure, the present invention integrated medical system may also handle more mundane aspects of accounting, patient history tracking, resource allocation, scheduling, record keeping, and security of information. Follow-up procedures can also be made thorough and complete. The architecture is flexible to permit the integrated medical system to function in any type of a medical facility ranging from a dispensary to a complete hospital and health care complex.

The methodology upon which the integrated medical system functions is by partitioning the procedural steps in their modular microscopic or macroscopic forms. Logical relations between these steps permit their programming. Weak logical relations demand more intricate programs invoking additional searches or knowledge-based functions. Weak logic, coupled with uncertainty, reduced the confidence to an extent that the integrated medical system turns the problem to a group of on-line physicians.

One situation where human intervention is usually not needed is the communication network. Calls from the subscribers are routinely processed by telecommunication networks and special service oriented calls are processed by intelligent networks. These networks systematically reduce the switching, transmission, and administration functions of "call processing" in a series of machine executable subfunctions and are completed in an orderly way depending upon the outcome of the previous process. The call is initiated, processed, monitored, and completed over the duration of the call. This may take several seconds (voice calls) to several months (dedicated lines) or even a life-time (700 numbers operations) depending upon the service required. In this situation, dealing with communication networks, the deeper insight and the superior perception of the human is not necessary for the "call-processes" which in the decade of nineties are well streamlined and optimally programmed.

One situation where the human intervention is constantly needed is the maneuvering of an airplane or a spacecraft. In this case, the input conditions are so various that the human intelligence is likely to produce significantly improved results. In addition, the cost of failure is very high. The invocation of human insight and perception greatly influences the output and reduces the probability of fatal errors.

In the case of the present invention integrated medical computer system, the parameters lie in a range somewhere between the two extreme examples given above. At the two extreme blends of man-machine team, the environment may be adjusted sufficiently to generate an all-man-no-machine hospital or an all-robot-no-man hospital. Through the present invention integrated medical computer system, the less expensive mechanized computer systems can take over some of the functions performed by the more expensive physician teams, thus generating more economical and optimal medical environments in countries where physicians and their administrators are expensive.

### II. Processor Environments in Integrated Medical System

In the present invention system, the architecture of the control processing unit of any moderate speed computer is modified with enhanced memory capabilities to process the medical procedures for patients. The enhanced memory will hold executable programs for the patient in the special hospital, medical center, or even the country in which the procedure is going to be performed. These subprocedures will be intelligently assembled based upon the artificial intelligence oriented knowledge bases. For this reason the high level language software (the compiler-assembler-loader-linker software chain) of the integrated medical system to be significantly different from that of the traditional computer systems. Thus, the set of assembly level procedural instructions will be tailored to the specific medical facility, thus calling for a facilities dependent assembler. This type of adaptation is routinely made for computer systems at installation time depending upon the hardware in that particular system. It is also done for the switching systems when any software controlled switch (e.g. electronic switching system facility) for any telecommunication system is installed.

The dependability concern in present invention systems may be handled by dual processors and elaborate error checking. The error checks not only verify the processing, but also the validity of the results based upon the artificial intelligence based expected outcome. Any unexpected findings are referred to the physician team on duty. Results of procedures will be entered into a patient database. Opinions and subjective comments will be entered onto a voice activated message retrieval system. Pictures, X-rays, and CATSCANs may be entered onto a visual database of the computer system, making the integrated medical system a truly multi-media system.

### III. Medical Computer Architecture

### 3.1 The Medical Processor Unit

Traditional control processing unit architectures entailing the control unit, the arithmetic unit, the logic unit, the control memory, interrelated with single or multi bus structures simply do not suffice for the present invention medical applications. In the present invention, medical processor unit does not process data. It only generates a sequence of subprocedures for current or standard medical procedures consistent with the patient history, physician's - expert system capability/expertise, and capability of the medical facilities available in that particular environment.

The typical operator-operand function of the medical processor unit of the present invention is neither traditionally binary nor hexadecimal; instead it is based on a combination of factors, such as the knowledge of the patient (available in the patient database), and the experience of the medical team, in a general categorical sense and with reference to that particular patient. Both of these are dynamic and can vary significantly. For this reason, expert and knowledge system-based programs are used and used consistent with the capability of the particular medical facilities which will be utilizing the present invention system.

The microprograms in the medical processor unit will function in three independent fashions initially and then in an interdependent fashion subsequently. First, any approved expert system database is referred for current concepts and breakthroughs. Second, the practice of the particular physician and the medical facility is referred to in order to be consistent with the knowledge level of the physician and the medical facility. Third, experience of this physician and earlier physicians with this particular patient is referred. All the necessary checks and verifications are made before administering any particular procedure to any particular patient in view of the patient history.

The output of the present invention system medical processor unit is a series of instructions based upon the opinion of the current experts approved by a team of physicians (if necessary) which has been verified for processing and expectation by dual independent processors. The output of the processor is a series of subprocedures which will be dispatched to the actual medical facilities after appropriate allocation of existing medical resources to the patient's subprocedures. Scheduling, resource sharing, time and priority allocation will be done automatically depending on the time or code of service being performed (emergency, standard, diagnostic, routine, etc.). This system is accountable for every step of its function and no tampering of any sort is possible by encryption of subprocedural code and patient data. The decryptor key and its code identification is available to the medical staff. Such measures of additional safety and streamlined handling of the procedures can make the overall service provisioning current, economic, and accountable at every subprocedural step.

### 3.2 Subprocedure Instructions

From preliminary considerations, these may be numerous broadly definable instructions. Three such categories are knowledge based inferential instructions, search instructions, and administrative or local housekeeping instructions.

In the first category, the knowledge structure of the cause-effect relation is queried, or the logical predictable effect is being sought, or conversely the preconditions for a given state is being investigated. It is important to note that certainty is never the strong point of this type of processing. Every step is prone to a confidence level. Thus the chain of subprocedures which make up a given procedure is the product of such individual confidence levels. Fuzzy-set information processing is most appropriate for the execution for this category of instructions.

In the second category, the search is being initiated. Two groups emerge as follows. The first group deals with search in the knowledge (professional, medical opinion, possible cures, diagnostics, etc.) domain and the second group deals with search in the data (files, patient information, databases, insurance company codes, service providers, drug vendors, etc.) domain.

In the first group, the input is the incomplete input data to investigate which other twigs of the knowledge tree are logically associated with the given inputs. Forward and backward pointers may be sought by this type of instruction. Similar symptomatic conditions may be queried. Associative and forward searches may be initiated by this type of instruction. The first category and second set of instructions are not always orthogonal and involving the first set can also invoke the second set and vice versa. Recursive searches should then be possible to/from and within the knowledge domain linking the objects in the data domain.

In the second group, the input is complete for the data processing units to offer complete and definite response. Example of this type of search is the social security number of a patient or the last visit to the hospital. Definite and precise answers are sought and secured from the present invention integrated medical system. By separating the searches in two groups, the instruction sets to the integrated medical system can be separated as (a) searches dealing with the knowledge domain or (b) searches dealing with the data domain. The hardware, software, firmware, and peopleware domains responsible for the execution of these two types of searches may thus be isolated and optimized.

In the third category, the administrative functions of the local medical facility may be activated. Typical of such instructions are scheduling of operating rooms, issuance of the hospital beds, allocation and consistency of the physician teams, accounting and billing, updating of the patient databases, etc. This category of instruction is localized to the environment of the medical facilities, the personnel, the support staff, and the type of services it is expected to provide. It is suggested that the input/output bus for this category of instruction be isolated from the input/output bus for the other two types of instruction to avoid any possible contamination of information to and from the established knowledge bases. Standard techniques for the construction, maintenance, and use of such knowledge bases are available.

### 3.3 Speed and Capacity of the Medical Processor Unit

From the point of view of implementation, the speed of the medical processor unit is not crucial, except when the physician prefers a real-time look up of how the medical processor unit would handle a certain situation. For this reason we foresee two types of medical processor units. The first type of medical processor unit handles the batch type of job processing and the second type handles the on-line processing. The architecture of the two systems thus differs.

### 3.3.1 Remote Medical Processor Units (Job Processing Environments)

Under normal conditions any particular patient's identification may be submitted to the medical processor unit with a desired procedure or even a complaint or symptom and the primary medical/vital data of the patient (e.g., temperature, blood pressure, heart rate, etc. as the paramedical teams dispatch to the physician). The non real-time response will be generated and mailed (dispatched electronically) to the doctor. Under the latter conditions, the memory and database capability of the medical processor unit to act in conjunction with the artificial intelligence based programs becomes the key element. Typically, in remote medical processor unit's, the memory size and database capability can be compromised for speed. Dual independent processing (as it is done in most electronic switching system facilities) can only double the cost of high capacity low-speed machine. Job priorities are assignable to the patient and procedures. Procedure sequences are forced in subsequent time slots and time and resource sharing can thus be optimized.

Almost all remote medical processor units need to be network based since the medical knowledge bases can also be remote and one medical processor unit can service many hundreds of remote input locations. The searches performed by remote medical processor units can become more thorough and wide in non real-time applications.

### 3.3.2 Hospital Based Medical Processor Units (On-line Processing)

When real-time response is necessary for the medical processor unit, architecture can be readjusted for speed and response rather than extensive searches for a large number of patients and subscribers. When certain procedures are necessary quickly, cost of service provisioning, and economics of providing services from various hospitals, laboratories, diagnostic services, or even doctors may be sacrificed for urgency. However, under normal conditions, the patient can be provided with the most cost effective strategy for the services to be performed. Specialized service providers will effectively use all their resources optimally and marginal service providers will get phased out.

### 3.3.3 Processor Speed, Confidence level and Patient Load

The most time consuming task of the processor is the search through numerous knowledge bases to achieve a high confidence in the inference it draws about the next logical step or the next inferential step. One can defeat the integrated medical system by insisting upon a 100 percent confidence with insufficient input data with exhaustive knowledge bases or conversely with sufficient input data but with inadequate knowledge bases. To be realistic about the results that the integrated medical system can generate, a compromise in what is being queried is essential. Being a programmed system, it can provide only inferential results with varying degrees of confidence. Exhaustive searches take a longer response time as do the peak-hour high-patient-load queries. Here the processor speed provides a compromise. Expensive high speed systems can yield high confidence inferences at the busiest hospital hours and vice versa. Smaller medical facilities will thus need a lower power processor. Job scheduling algorithms by the operating system (as they are used in traditional computer systems) will prove useful in generating an acceptable response time from the integrated medical system.

Other tasks such as patient scheduling, shared resource allocation, sequentiality of subprocedures, minimum hospital stay requirements, etc. are relatively trivial for the integrated medical system.

### IV. Architectural Considerations

### 4.1 Processor-Based Local-knowledge-Bank System

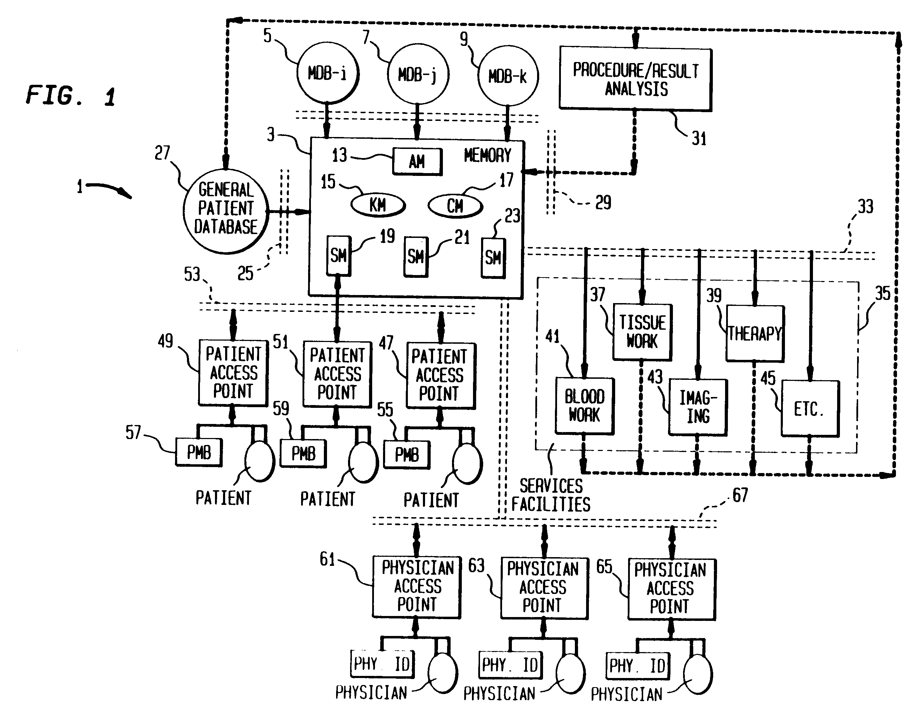

Figure 1 shows a processor-based local knowledge bank integrated medical computer system 1. Here, the processor 3 and knowledge banks are in close proximity such that bus lines can be extended from processors to these knowledge banks (medical data banks 5, 7 and 9). The addressing is done via bus-selector through bus 11 tied to a particular knowledge bank. The address of the bus may be consistent with the classification of the information stored in that particular bank thus reducing the seek time in these massive information stores. In such systems, the instruction to the knowledge bank is followed by a burst of input data via a direct memory access channel. Note that the contact between the patients and the physicians may be in real-time, or via remote access.

Considerable latitude exists in the design of the memory, processor, and the bus structures. At one extreme, we have the knowledge banks bursting their entire segments of all the relevant information back to the main memory of the medical processor 3 where the instruction is executed. At the other extreme, we have a complete instruction being dispatched by the medical processor 3 to the knowledge banks and the knowledge banks, e.g., in their own local processors execute instructions or part thereof. The partial result (in a shorter burst) of the instruction is dispatched back to the medical processor 3.

This aspect of the processing is novel over the conventional computing environments where part of the execution of the instruction takes place in the memory. Some of the sophisticated database software packages can perform these functions for the integrated medical system. The compromises in the cost and performance are evident from the two hardware configurations.

Every subprocedure is thus executed and the net result of the procedure is conveyed to the user (or the user program). The output is generated from subprocedures, procedures, runs, and entire usage of the integrated medical system in an orderly and systematic fashion. Debugging of the integrated medical system functions becomes as easy as reading the registers and core dump of the medical processor unit or the registers and the core dump of the local processing units of the knowledge banks.

The processor 3 includes a memory and an administrative module 13, a knowledge module 15, a communications module 17 and switching modules 19, 21 and 23. In addition to the knowledge bus 11, there is a patient bus 25 connected to general patient database 27, a procedure/lab bus 29 connected to a procedure/result analysis 31. Output bus 33 is connected to services facilities 35, which may, for example, include tissue work 37, therapy 39, blood work 41, imaging 43, and others (etc.) 45, such as, for example, urinalysis.

Processor 3 is also connected to a plurality of patient access point units 47, 49 and 51 via input bus 53. These units can include individual patient medical bases 55, 57 and 59 and can include input to processor 3 as well as output from processor 3. These units could monitor patient's conditions, input data, provide instruction to the patient and track patient conditions and alert the hospital staff and/or physician(s) to such changes.

There may also be connected to processor 3 a plurality of physician access point units 61, 63 and 65, via physicians bus 67. Though these units physicians may access services facilities 35, procedure/result analysis 31, any or all medical data banks 5, 7 and 9, general patient database 27 and patient accesspoint units 47, 49 and 51. The physicians may do so through processor 3 as a direct user via such buses described above, or indirectly by query to the processor 3, whereby the processor 3 will utilize whatever modules, memory, programs, subprograms, buses and connected units and facilities as may be necessary or appropriate to respond to particular queries posed by the physician user.

### 4.2 Distributed-Knowledge-Bank System

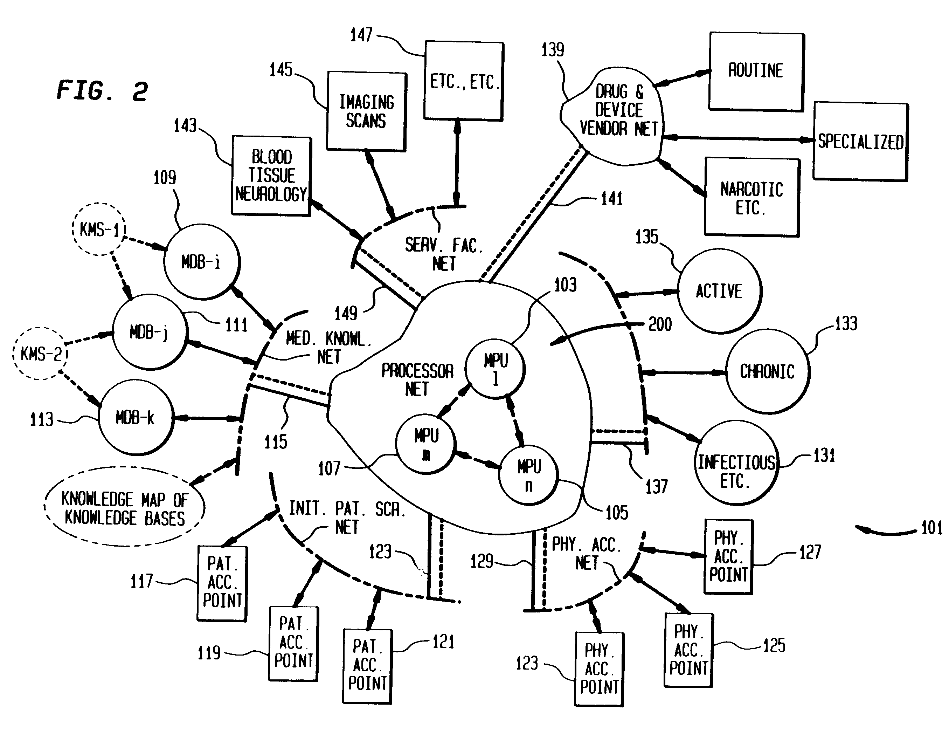

In this type of present invention system 101 shown in Figure 2, many medical processor units e.g. units 103, 105 and 107 and knowledge banks 109, 111 and 113 are linked via a high speed integrated services digital network 115. Isolated packets of information arrive at the knowledge banks from numerous integrated medical systems connected therewith. Thus, the collection of numerous units, such as units 103, 105 and 107 form a processor net 200. Optimal protocol design and packet structure is a matter of design by the artisan now that this environment has been defined. For example, the addressing of the distant knowledge banks is done via a subject matter identifier allocated to the distant knowledge bank. This identifier of the knowledge bank is consistent with the information stored in that particular bank thus reducing the switching time to these massive information stores. In such systems, the instruction to the knowledge bank is followed by a burst of input data via the packet switching network located within processor net 200. (It should be assumed that processor net 200 contains individual processors similar to processor 3 of Figure 1 with the memory and various modules as described therein.)

Every subprocedure is thus executed via an individual packet command (like the modern signalling system, packet commands or subprocesses in the back-bone network are embedded in the intelligent networks). The net result of the procedure is conveyed to the user (or the user program) by a series of packet transactions. Such transactions between a single integrated medical system and multiple knowledge banks are systematically processed and the output is accumulated from subprocedures, procedures and runs. The entire usage of this present invention network based integrated medical system is as orderly and systematic as job processing in distributed computer environments.

Referring again to Figure 2, the integrated medical system 101 may not only include integrated services digital network 115 for connection to knowledge banks (medical data banks), but may have a plurality of integrated services digital networks for various predetermined functions. Thus, integrated services digital network 123 is connected from net 200 to patient access point units 117, 119 and 121; integrated services digital network 129 is connected from processor net 200 to physician access point units 123, 125 and 127. Likewise, net 200 is connected to general patient databases, such as infectious diseases 131, chronic illnesses 133 and active ailments 135, via integrated services digital network 137; is connected to drug and device vendor net 139 via integrated services digital network 141; and is connected to service facilities net functions such as blood, tissue and neurology 143, imagining scans 145 and other functions 147, via integrated services digital network 149.

Debugging of these types of integrated medical system functions becomes as easy as studying the packet contents of any given procedure.

### V. Input/Output Considerations

### 5.1 Input Aspects

Compared to conventional computer system/networks, the input/output interface parameters can be highly variable for any of the present invention systems. Streamlining the human and verbal communication may be somewhat effective to accelerate input, but perfection is impossible. For this reason, it is proposed that, in preferred embodiments, the input to present integrated medical systems be via laser or otherwise encoded credit-card-sized medical identification for routine cases (or a sequence of cards for extremely acute cases). A short data structure of vital statistics, chronic conditions, allergies, blood type, genetic predispositions, etc. may be encoded on this card with a forward pointer to where (i.e., patient database location) all of the patient history is stored. The input processors may be located like credit card centers or automatic bank teller machines throughout the population centers tied to the integrated medical system.

It is desirable to have a progression of such medical card centers ranging from most rudimentary to sophisticated medical centers. Typical examples of the rudimentary facilities are those for dispensing routine medication (like insulin or hypertension medication, or birth control pills, etc.) from integrated medical system controlled medication warehouses. It is expected that the integrated medical system will make an appointment for the patient every so often after the programmed sequence of refills is complete and direct the patient to go to next hierarchical medical card center.

Typical of the next hierarchical medical card center is one equipped with other monitoring devices (such as those for reading the temperature, blood pressure, throat scanners, EKG recorders, imaging centers, etc.). It is also expected that the most recent patient input is entered into the patient database via the integrated medical system. Whereas the higher level centers can perform the functions of the lower level centers, they still act as a check point to the access of the highest levels of the medical card centers.

At the highest level of the input processors exist the current staffed hospitals, therapy centers, delivery rooms, operating theaters, intensive care facilities, etc. The situations requiring the services of these facilities typically defy the programmability of the next step. However, since the integrated medical system is networked to service all medical card centers, access and referral throughout card centers on the network is automatic.

### 5.2 Output Aspects

The output from the integrated medical system can also have significant variations. Ranging from no action to immediate hospitalization or surgery, the integrated medical system reaches all medical service facilities such as drug dispensing machines, physical therapy centers, blood banks, nursing homes, and the higher levels of the medical card centers listed in Sec. 5.1.

One of the possible ways of handling the outputs of the integrated medical system is via specialized nodes created on the network. Similar to the knowledge processing where nodes are addressed by the subject matter they hold, the output nodes are addressed by the type of service that is scheduled through them. For example, if the architecture is equipped via a node for physical therapy all requests for this service would go through this node. The geographical dispersion of the patients and services center is tackled by mapping the zip codes of the patient and of the service provider. One example where such service is already provided is by matching the real estate agent with clients moving into a new area.

### 5.3 Overall Input/Output Design

If the medical processor unit is considered as a single entity, it is logical in many environments to isolate the input and the output bus of the medical processor. System dependability is greatly enhanced. Since the integrated medical system is generally network based, the separation of the input and output ports on all the medical processor units in the network would be desirable to reduce any risk of malfunction or logical processing the inputs and coordinating the corresponding outputs.

If the argument for the separation of the input and the output ports is taken one step forward, then it is easy to see the input/output architecture of any medical processor unit. Inputs from numerous medical card centers will follow the input path to most logically accessible medical processor unit via the type of medical service request. If certain medical processor units can handle specialized services (depending upon the contents of its own knowledge banks), then the logical address of the medical processor unit is determined by the network by mapping the service request for the patient with the service capability the medical processor unit is specially capable for handling. The type of hierarchical addressing reduces the complexity of the look-up tables for the particular medical processor unit and the service provider for any service request for any patient.

The specialization of the medical control units, and that of the service providers in by itself thus provides the logical addressing of these nodes in the integrated medical unit. It is expected that if the integrated medical system architecture is designed based upon the separation of specialty, then the separation of the input and output ports of the individual medical control units, then the flow of information can be significantly streamlined thus reducing the probability of any possible errors in processing the medical information for any particular patient.

### VI. Implementational Considerations

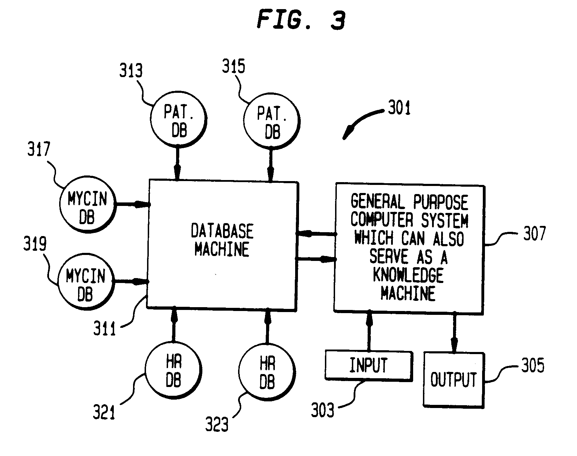

At first level the integrated medical system 301 may be fabricated by a two (or a network) of general purpose computers (see Figure 3) with one knowledge machine 201 acting as the execution unit of the knowledge-based subprocedural instructions such as the processor 3 of Figure 1, or a subset of the functions of processor 3 of Figure 1. (Reference is made to the first and second categories of instructions in Sec. 3.2 above). At the most rudimentary level of software, standard packages such as MYCIN or INTERN may be used in the knowledge machine 201.

Any special hardware or system, functioning as a database machine 311 holds at least a subset of the medical knowledge for a subspecialty. Access to patient databases 313 and 315, MYCIN databases 317 and 319 and hospital resource databases 321 and 323 would be a reasonable first level connection. The language and user interface input 303 can be made keyboard or mouse driven. The output 305 can be made displayed on rudimentary graphic displays. A single user system greatly reduces the need for any sophisticated or expensive hardware.

The housekeeping functions may also handled by one general purpose computer hardware unit within the knowledge machine 307 with a special software for resource (computer and medical) allocation, billing and patient record keeping. This is typical of a very small computer system specifically tailored for any given application.

With some initial software organization and debugging, these rudimentary medical systems can become functional in a very short time. For the implentation of large integrated medical systems, specialized software deployment strategy will become essential for optimal performance, and to this extent the design of the integrated medical system is the similar to the design and deployment of any other large scale special purpose computer system with dominant component of knowledge processing and database capability. National and network access may be and preferably will be provided by the new communication protocol embedded in any intelligent network or even the upcoming integrated services digital network.

### VII. Machine/Network Failure and other Catastrophic Events

The probability of catastrophic failure is finite, even though it can be made less than any given smallest number by robust design. It is not our contention that such an event will not occur, instead we assert that certain routine precautionary steps in running and maintaining the integrated medical system can significantly reduce the collapse of the integrated medical system.

Some of these steps are similar to those taken by the Regional Bell Operating Companies in maintaining and safeguarding the Service Control Point database. Banks and computer systems routinely archive their information. Diagnostics are run frequently. Critical information is retained at more than one place. Constant copy and verify statements are executed in the system to monitor the accuracy and authenticity. Critical steps are run twice for confirmation. Incremental change of stored information is rechecked for accuracy and rationale. Dual processors are used to validate the processor functions. Error checking and correction is maintained in all transmission. Encryption is used to prevent tampering. Physician and technician code are checked for authorization to modify or alter the medical records before any change is accepted.

Precautionary measures are based upon the possible reasons and sources of errors. In large, computer systems security issues have been tackled to make them less error prone. In the two architectures suggested, only the modules which do interact are interconnected. Logical and access (by both hardware and software techniques) denial block contamination of information due to processor or software error or to some extent the hardware malfunction. The architectures proposed permit some capacity to identify and isolate errors and the modules in which they start. The system monitors these conditions before a catastrophic mishappening by blocking erroneous hardware from its communication capability.

Present invention integrated medical computer systems are likely to foster over- dependability upon itself. Vigilance may sometimes be lost and people may tend to become addicted to the integrated medical system capability and power. These issues may persist in any situation where new systems are introduced. Corporations may become addicted to their management information systems, banks can not function without the network response, stock markets can not transact when the network is down, and so on. Transition to integrated medical system is likely to meet a considerable negativism like management information system has met in corporations and robotics has met in mass production. However, not to bring the true capabilities of computers, networks and artificial intelligence techniques to medicine appears to be like withholding calculators from schools.

### VII. Conclusions

Numerous versions of the present invention integrated medical computer systems have been presented. The classification of subject matter and the design of modular software oriented to the performance of medical subprocedures becomes essential. Coordination of these medical subprocedures with the expertise of the medical staff and the use and availability of medical facilities in the hospital or the medical center becomes a crucial step in the deployment of the integrated medical system in practice.

The separation of the input and output processing at the nodes of the medical processor units in the network is desirable to streamline the multitude of input-output transactions at any integrated medical system node. The hierarchical separation based upon the specialization of the medical processors makes network addressing of these nodes flexible and straight forward. Additional nodes may be added or removed from the integrated medical system. The hierarchical separation based upon the services provided by the service providers also makes the network addressing of these vendors flexible, straight-forward and programmable. Such vendors may be added or deleted from the integrated medical system to offer most optimal and economical service by the integrated medical system.

This approach is feasible in light of the recent developments in database storage and access technology (for the hierarchical organization of medical information, its real-time access, and associative searches). Artificial intelligence techniques (for knowledge processing such as forward/backward linking, inductive and predictive logic, pattern recognition, expert system consultation capability), and specialized operating systems (for resource management, allocation, conflict and dead-lock resolution, accounting, billing and record keeping). With the cooperative role of the interdisciplinary team of systems designers, integrated medical system can be as useful to the health industry as the airplanes are to the transportation industry or as computers are to the scientific community.

Obviously, numerous modifications and variations of the present invention are possible in light of the above teachings. It is therefore understood that within the scope of the appended claims, the invention may be practiced otherwise than as specifically described herein.

## Claims

1. A hospital-based integrated medical computer system for processing medical and patient information and for evolving medical knowledge, diagnoses and prognoses which comprises:
(a) at least one processor, including a memory and a plurality of medical data banks connected thereto, and a plurality of separate processor hardware modules at least indirectly connected to said memory, said modules including a communication module, at least one switching module, an administrative module and a knowledge base module;
(b) hardware, firmware and software in said processor hardware modules to enable the modules to perform at least the following functions:
(i) for the communication module, to control all functional processes of the other modules, the main memory and said at least one processor, so that they effectively communicate with one another;
(ii) for said at least one switching module, to select and switch between selected information as it becomes relevant to a process of solving a particular problem;
(iii) for the administrative module, to perform housekeeping functions, including multitasking control with resource allocation, real-time multitasking and scheduling of tasks; and,
(iv) for the knowledge base module, to operate knowledge processing functions and to store information in said medical data banks.

2. The hospital-based integrated medical computer system of claim 1 wherein said system further includes a general patient database connected to at least one of said memory and said processor hardware modules.

3. The hospital-based integrated medical computer system of claim 1 wherein said system further includes a plurality of physician-based, remotely located physician access point units connected to said processor for operating said system from remote sources.

4. The hospital-based integrated medical computer system of claim 1 wherein said medical data banks include a plurality of subareas of knowledge, and said knowledge base module includes a plurality of subprocesses, and said communication module has software to further control communication between the subareas of the medical data banks and the knowledge base module and to control the subprocesses as they pertain to a particular medical problem and to systematically organize inputs and outputs of the subprocesses.

5. The hospital-based integrated medical computer system of claim 1 wherein said system further includes a plurality of remotely located patient access point units connected to said processor for operating said system from remote sources by patients for predetermined purposes.

6. A method of operating a hospital-based integrated medical computer system for processing medical and patient information and for evolving medical knowledge, diagnoses and prognoses which comprises:
(a) operating at least one processor, which includes a memory and a plurality of medical data banks connected thereto, and a plurality of separate processor hardware modules at least indirectly connected to said memory, said modules including a communication module, at least one switching module, an administrative module and a knowledge base module;
(b) operating hardware, firmware and software in said processor hardware modules to perform at least the following functions:
(i) for the communication module, controlling all functional processes of the other modules, the main memory and said at least one processor, so that they effectively communicate with one another;
(ii) for said at least one switching module, selecting and switching between selected information as it becomes relevant to a process of solving a particular problem;
(iii) for the administrative module, performing housekeeping functions, including multitasking control with resource allocation, real-time multitasking and scheduling of tasks; and,
(iv) for the knowledge base module, operating knowledge processing functions and to store information in said medical data banks.

7. The method of claim 6 wherein said system further includes a general patient database connected to at least one of said memory and said processor hardware modules.

8. The method of claim 6 wherein said system further includes a plurality of physician-based, remotely located physician access point units connected to said processor for operating said system from remote sources.

9. The method of claim 6 wherein said medical data banks include a plurality of subareas of knowledge, and said knowledge base module includes a plurality of subprocesses, and said communication module has software to further control communication between the subareas of the medical data banks and the knowledge base module and to control the subprocesses as they pertain to a particular medical problem and to systematically organize inputs and outputs of the subprocesses.

10. The method of claim 9 wherein the software for the communication module includes the ability to select and collate subprocesses of the knowledge base module and the data from the medical data banks towards solving a particular medical query in a perceptive manner.
